# EUROPEAN PATENT SPECIFICATION

(11) **EP 4 282 856 B1**
(45) Date of publication and mention of the grant of the patent: **10.09.2025**
(21) Application number: 22899903.3
(22) Date of filing: 07.07.2022
(51) Int. Cl.: C07C 309/14, B01J 31/18, C07C 303/32, C07C 303/44

(54) **METHOD FOR PREPARING TAURINE**
VERFAHREN ZUR HERSTELLUNG VON TAURIN
PROCÉDÉ DE PRÉPARATION DE TAURINE

(30) Priority: 30.11.2021 CN 202111446352
(43) Date of publication of application: 29.11.2023
(73) Proprietor: Zhejiang Nhu Pharmaceutical Co. Ltd., Shaoxing, Zhejiang 312369 (CN); Shangyu Nhu Bio-Chem Co., Ltd., Shaoxing, Zhejiang 312369 (CN); Zhejiang NHU Company Ltd., Shaoxing, Zhejiang 312500 (CN)
(72) Inventor: XU, Songhua, Shaoxing, Zhejiang 312369 (CN); YAO, Xianghua, Shaoxing, Zhejiang 312369 (CN); SONG, Yinjiang, Shaoxing, Zhejiang 312369 (CN); HE, Xiaoxiang, Shaoxing, Zhejiang 312369 (CN); CHEN, Lihuan, Shaoxing, Zhejiang 312369 (CN); LI, Feng, Shaoxing, Zhejiang 312369 (CN); PAN, Yinxia, Shaoxing, Zhejiang 312500 (CN); ZHAN, Pingping, Shaoxing, Zhejiang 312500 (CN)
(74) Representative: karo IP
(86) International application number: PCT/CN2022/104337
(87) International publication number: WO 2023/098083

(56) References cited:
- CN-A- 108 329 239
- CN-A- 108 329 239
- CN-A- 110 452 136
- CN-A- 110 903 222
- CN-A- 111 269 151
- CN-A- 111 320 558
- CN-A- 111 574 412
- CN-A- 111 574 412
- CN-A- 112 979 505
- CN-A- 113 061 101
- CN-A- 114 349 666
- US-A1- 2019 135 739

## Description

### TECHNICAL FIELD

The present disclosure belongs to the field of preparation of medical intermediates, and specifically relates to a method for preparing taurine.

### BACKGROUND

Taurine (2-aminoethanesulfonic acid), also known as taurocholic acid or taurobilin, is a white crystal or powder, which is odorless, non-toxic and slightly sour. As a non-protein amino acid, the taurine is one of essential amino acids in the human body, and has unique pharmacological and nutritional health care functions. The taurine can be widely used in medicine, food additives, fluorescent brightening agents, organic synthesis and other fields, and can also be used as a biochemical reagent, a wetting agent, a buffer and the like. The taurine has been widely used in medicine and food additives in western developed countries.

Chemical synthesis methods of the taurine mainly include an ethanolamine method and an ethylene oxide method. According to the ethanolamine method, ethanolamine is used as a raw material, and the taurine is synthesized in two steps. However, an esterification reaction is a reversible reaction, which is difficult to conduct completely, so that the conversion rate of the ethanolamine and the reaction yield are restricted. Moreover, sodium sulfate is generated in the reaction system, so that difficult separation is likely to be caused, the yield and quality of a product are affected, and the environmental pressure is great. According to the ethylene oxide method, ethylene oxide is used as a raw material to undergo a ring-opening addition reaction with sodium sulfite first, followed by a reaction with ammonia under heating and pressurizing conditions to synthesize sodium taurine, and acidification is conducted to obtain the taurine. By using the method, the raw material is cheap and easy to obtain. Thus, compared with the ethanolamine method, the method has an obvious cost advantage, and the reaction yield is also slightly higher than that of the ethanolamine method.

The key step of the ethylene oxide method is to prepare sodium taurine by an ammonolysis reaction of sodium hydroxyethyl sulfonate and liquid ammonia. In initial studies, such as in a patent US1932907, sodium hydroxyethyl sulfonate undergoes a reaction with ammonia at a temperature of 240-250°C without adding a catalyst. Moreover, the yield of resulting sodium taurine is only 80%, the production cost is high, and reaction conditions are harsh. According to a patent DD219023, sodium sulfate and sodium hydroxide are added as catalysts for a reaction at 280°C for 45 min to obtain an ammonolysis product including 71% of sodium taurine, 29% of sodium distaurine and sodium tristaurine, and many by-products. It can be seen that the sodium taurine is an important intermediate in preparation of the taurine by the ethylene oxide method. The yield and purity of the taurine are determined by the conversion rate and selectivity of the sodium taurine produced by the ammonolysis reaction of the sodium hydroxyethyl sulfonate.

According to a patent CN105732440A, any one or a combination of sodium carbonate, potassium carbonate, iron/aluminum metal salts, NiO/CeO₂, rare earth oxides and other catalysts is added as a catalyst in the process of an ammonolysis reaction including application of a mother solution, and the yield of sodium taurine can be increased to 90-95% after circular application. However, the one-way yield is not mentioned. In addition, reaction conditions are still harsh, the reaction is carried out at a temperature of 255-265°C and a pressure of 19-20 MPa.

According to a patent US20160355470A1, it is disclosed that in an ammonolysis reaction of sodium hydroxyethyl sulfonate, the addition of NaOH can catalyze the ammonolysis reaction in the direction of generating sodium taurine. By means of the ammonolysis reaction, the yield of the resulting sodium taurine can be greater than 90%, but the ammonolysis reaction still has many by-products. Meanwhile, the reaction still requires harsh conditions including a high temperature of 160-260°C and a high pressure of 26 MPa.

According to patents CN111269151A, CN111689880A and CN111574412A, Zr₂MoO₄(PO₄)₂, Pd-C/Al₂O₃ selective catalyst, BaCaTiO₄ and other types of heterogeneous catalysts are used as a catalyst for ammonolysis. Compared with conventional alkaline catalysts, these catalysts require milder reaction conditions and shorter reaction time. However, the catalysts are too complex to prepare, so that the production cost is increased.

CN 108 329 239 A provides a method for preparing taurine from sodium isethionate. A molybdate homogeneous catalyst is adopted for catalyzing ammonolysis reaction of sodium isethionate to obtain a finished taurine product through steps of neutralization, crystallization separation and the like. Compared with a traditional alkaline catalyst, the process has advantages that the temperature and pressure of ammonolysis reaction of sodium isethionate can be remarkably reduced, reaction time is shortened, and easiness in industrial production is achieved.

In summary, a more efficient method for preparing sodium taurine is required, so as to improve the yield and purity of a sodium taurine product. Meanwhile, reaction conditions are as mild as possible, so as to reduce the energy consumption and the production cost in a production process.

### SUMMARY

In view of the above problems, the present disclosure provides a method for preparing taurine. The method has the advantages of mild process conditions, short reaction time, high yield and a simple catalyst source.

In order to solve the above problems, the following technical solutions are adopted by the present disclosure.

A method for preparing taurine includes the following steps:
(1) subjecting a sodium hydroxyethyl sulfonate aqueous solution to an ammonolysis reaction with ammonia under the action of an ammonia water complex ion catalyst to obtain a reaction solution containing sodium taurine; and
(2) subjecting the reaction solution containing sodium taurine to deamination, dewatering by concentration, acidification and crystallization to obtain the taurine and a mother solution;

where the ammonia water complex ion catalyst is obtained by complexation of ammonia and a catalyst precursor, and the catalyst precursor includes one or more metal elements of chromium, zinc, nickel, cobalt, copper and silver;
wherein in step (1), a method for preparing the ammonia water complex ion catalyst comprises: adding the catalyst precursor to ammonia water, and conducting stirring for dissolution to obtain the ammonia water complex ion catalyst;
and after the ammonia water complex ion catalyst is obtained, the sodium hydroxyethyl sulfonate aqueous solution and liquid ammonia are added for the ammonolysis reaction.

In the present disclosure, the novel ammonia water complex ion catalyst is used for catalyzing the ammonolysis reaction. The catalyst has high reaction activity, so that the ammonolysis reaction has mild reaction conditions, low reaction temperature and pressure, short reaction time and high reaction yield.

As a preference, in step (1), the catalyst precursor is a metal elemental, a metal oxide or an organometallic salt.

As a preference, in step (1), the catalyst precursor includes one or more of chromium trioxide, a zinc powder, nickel acetate, cobalt acetate, copper oxide and silver oxide. A better catalytic effect can be achieved by using the catalyst precursor.

A method for obtaining the ammonia water complex ion catalyst of the present disclosure is simple. As a preference, in step (1), a method for preparing the ammonia water complex ion catalyst includes: adding the catalyst precursor to ammonia water, and conducting stirring for dissolution to obtain the ammonia water complex ion catalyst; where the concentration of the ammonia water used is 10-30%;
and after the ammonia water complex ion catalyst is obtained, the sodium hydroxyethyl sulfonate aqueous solution and liquid ammonia are added for the ammonolysis reaction.

Preferably, in step (1), the ammonia includes one or two of ammonia water and liquid ammonia. Further preferably, the ammonia is a combination of ammonia water and liquid ammonia, the ammonia water is ammonia water remained in the preparation process of the ammonia water complex ion catalyst, and the liquid ammonia is newly added liquid ammonia.

Preferably, in step (1), the use amount of the ammonia water complex ion catalyst is 0.5-8 wt% of the sodium hydroxyethyl sulfonate, preferably 1.0-4.0 wt%, and more preferably 1.2-2.0 wt%, where the use amount of the ammonia water complex ion catalyst is calculated based on the added catalyst precursor.

Preferably, in step (1), the concentration of the sodium hydroxyethyl sulfonate is 10-45 wt%, preferably 15-45 wt%, and more preferably 35-45 wt%.

Preferably, in step (1), the concentration of the ammonia in the reaction system is 10-30 wt%, preferably 15-27 wt%, and more preferably 17-22 wt%.

In step (1), the ammonolysis is conducted at a temperature of 40-210°C, preferably 90-200°C, and more preferably 150-200°C.

In step (1), the ammonolysis is conducted at a pressure of 0.1-15 MPa, preferably 2-11 MPa, and more preferably 6-11 MPa. Unless otherwise specified, the pressure of the present disclosure is gauge pressure.

In step (1), the ammonolysis reaction is conducted for 10-120 min, preferably 10-80 min, and more preferably 20-60 min.

Preferably, in step (2), a flash evaporation method is used as a deamination method. Specifically, the excess ammonia remaining in the ammonolysis reaction in step (1) is removed by flash evaporation, and evaporation and concentration are conducted to increase the concentration of the sodium taurine to 18-25 wt%, preferably 19-21 wt%.

Preferably, in step (2), an acidifying agent for acidification is a weakly acidic resin for acidification. The use amount of the resin is about 0.5-1 time of the mass of the sodium taurine solution, and the use mount is preferably 0.7-0.8 time.

An acidification process using the resin includes allowing the obtained sodium taurine aqueous solution to flow forward through the weakly acidic resin first, stopping feeding when the pH of a material liquid at an outlet is close to 8.0, and then allowing deionized water to flow forward through the weakly acidic resin to precipitate the material liquid.

As a preference, the flow rate of the sodium taurine aqueous solution is 0.9-1.2 BV/h, and the flow rate of the deionized water is 1.5-2.0 BV/h. Preferably, in step (2), the crystallization is conducted by a cooling method; the crystallization is conducted at a temperature of 20-40°C, preferably 25-30°C; and the crystallization is conducted for 2-8 h, preferably 4-5 h.

In step (2), the mother solution can be recycled and applied to improve the use efficiency of the catalyst. A specific application method includes: concentrating the mother solution, and then mixing the mother solution containing the catalyst with ammonia water, liquid ammonia and a fresh sodium hydroxyethyl sulfonate aqueous solution for an ammonolysis reaction.

Preferably, the degree of concentrating the mother solution is that the total concentration of effective components such as sodium taurine, sodium ditaurine and sodium hydroxyethyl sulfonate in the mother solution after concentration is approximately equal to the concentration of the newly added sodium hydroxyethyl sulfonate aqueous solution.

Compared with the prior art, the present disclosure has the following positive effects.
(1) The catalyst has high catalytic activity, so that the ammonolysis reaction has milder reaction conditions, low reaction temperature and pressure and short reaction time.
(2) The generation of macromolecular by-products such as sodium ditaurine in the ammonolysis reaction is limited, the one-way yield of the sodium taurine can be obviously increased to 92-96%, and the conversion rate of the sodium hydroxyethyl sulfonate is obviously increased to more than 98%.
(3) After the catalyst in the mother solution is completely recycled and applied, the mother solution is uniformly mixed with the sodium hydroxyethyl sulfonate aqueous solution, the ammonia water and the liquid ammonia to undergo an ammonolysis reaction directly. The total yield of the taurine in the whole process can reach 92% or above, and the problem of three wastes is not caused.
(4) The catalyst can be obtained by a complexation reaction of the ammonia water and the cheap and easy-to-obtain catalyst precursor, the operation is simple, and the application is convenient.

### DETAILED DESCRIPTION OF EMBODIMENTS

A taurine intermediate and a method for preparing taurine mentioned in the present disclosure are further explained below in combination with examples.

Unless otherwise specified, all percentages referred in the following examples refer to mass percentage concentrations.

The taurine product of the present disclosure is qualitatively analyzed by comparing the residence time of HPLC according to a dansyl chloride derivation method.

### Example 1

200 kg of 25% ammonia water was pumped into a dissolution kettle, and 0.68 kg of a zinc particle catalyst was added and stirred for dissolution. 40 kg of liquid ammonia and 170 kg of a 40% sodium hydroxyethyl sulfonate aqueous solution were added and uniformly stirred to obtain a mixture. After being pressurized to 8 MPa by a high pressure pump, the mixture was heated to 200°C by a heat conduction oil preheater first, and then entered an ammonolysis reactor for a reaction. The ammonolysis reaction was conducted at a temperature of 200°C and a pressure of 8 MPa for 30 min to obtain a sodium taurine solution. Then, deamination and dewatering by concentration were conducted to obtain 317.86 kg of a sodium taurine aqueous solution. After sampling for detection, the content of sodium taurine was 20.0%, and the yield of the sodium taurine was calculated as 94.10%.

317.86 kg of the sodium taurine aqueous solution was allowed to flow forward through a 159 L weakly acidic 110 resin (English name: Amberlite^{®} IRC-76, and CAS number.: 11098-83-2) at a flow rate of 1.2 BV/h. When the pH of a material liquid at an outlet was close to 8.0, feeding was stopped. Then, the material liquid was washed with deionized water at a forward flow rate of 2 BV/h for recycling. The total mass of a collected material was about 831.92 kg, the content of taurine was detected as 6.5%, and the pH of the material liquid was about 7.0. 6.5% of the material was concentrated, cooled to about 25°C and crystallized to obtain 45.97 g of a taurine crude product with a mass content of 95%, a water content of 4.2% and an impurity content of 0.8%. A mother solution containing the catalyst was continuously concentrated to 40.80 kg, a fresh 40% sodium hydroxyethyl sulfonate aqueous solution was added to reach 170 kg, and then corresponding ammonia water and liquid ammonia were supplemented to conduct an ammonolysis reaction directly. Data of the application batch and the yield of an ammonolysis reaction are as follows.

| Application batch of a catalyst | Feeding mount of 40% sodium hydroxyethyl sulfonate | Feeding amount of a mother solution | Yield of an ammonolysis reaction |
|---|---|---|---|
| 1st batch | 170.00kg | 0kg | 94.1% |
| 2nd batch | 129.20kg | 40.80kg | 95.0% |
| 3rd batch | 128.20kg | 41.80kg | 93.9% |
| 4th batch | 129.50kg | 40.50kg | 96.5% |
| 5th batch | 129.70kg | 40.30kg | 95.3% |

### Example 2

250 kg of 25% ammonia water was pumped into a dissolution kettle, and 1.2 kg of a nickel acetate catalyst was added and stirred for dissolution. 30 kg of liquid ammonia and 200 kg of a 35% sodium hydroxyethyl sulfonate aqueous solution were added and uniformly stirred to obtain a mixture. After being pressurized to 6 MPa by a high pressure pump, the mixture was heated to 180°C by a heat conduction oil preheater first. Then the mixture entered an ammonolysis reactor for a reaction. The ammonolysis reaction was conducted at a temperature of 180°C and a pressure of 6 MPa for 45 min to obtain a sodium taurine solution. Then, deamination and dewatering by concentration were conducted to obtain 328.4 kg of a sodium taurine aqueous solution. After sampling for detection, the content of sodium taurine was 20.5%, and the yield of the sodium taurine was calculated as 96.8%.

328.4 kg of the sodium taurine aqueous solution was allowed to flow forward through a 192 L weakly acidic 110 resin at a flow rate of 1.1 BV/h. When the pH of a material liquid at an outlet was close to 8.0, feeding was stopped. Then, the material liquid was washed with deionized water at a forward flow rate of 2.1 BV/h for recycling. The total mass of a collected material was about 818.05 kg, the content of taurine was detected as 7%, and the pH of the material liquid was about 7.0. 7% of the material was concentrated, cooled to about 25°C and crystallized to obtain 47.32 g of a taurine crude product with a mass content of 94%, a water content of 5.0% and an impurity content of 1.0%. A mother solution containing the catalyst was continuously concentrated to 43.40 kg and directly applied in a fresh 35% sodium hydroxyethyl sulfonate aqueous solution, and then corresponding ammonia water and liquid ammonia were supplemented to conduct an ammonolysis reaction directly.

### Example 3

150 kg of 25% ammonia water was pumped into a dissolution kettle, and 0.82 kg of a chromium trioxide catalyst was added and stirred for dissolution. 40 kg of liquid ammonia and 100 kg of a 35% sodium hydroxyethyl sulfonate aqueous solution were added and uniformly stirred to obtain a mixture. After being pressurized to 10 MPa by a high pressure pump, the mixture was heated to 195°C by a heat conduction oil preheater first. Then the mixture entered an ammonolysis reactor for a reaction. The ammonolysis reaction was conducted at a temperature of 195°C and a pressure of 10 MPa for 30 min to obtain a sodium taurine solution. Then, deamination and dewatering by concentration were conducted to obtain 169.3 kg of a sodium taurine aqueous solution. After sampling for detection, the content of sodium taurine was 19%, and the yield of the sodium taurine was calculated as 92.5%.

169.3 kg of the sodium taurine aqueous solution was allowed to flow forward through a 92 L weakly acidic 110 resin at a flow rate of 0.9 BV/h. When the pH of a material liquid at an outlet was close to 8.0, feeding was stopped. Then, the material liquid was washed with deionized water at a forward flow rate of 1.8 BV/h for recycling. The total mass of a collected material was about 364.8 kg, the content of taurine was detected as 7.5%, and the pH of the material liquid was about 7.0. 7.5% of the material was concentrated, cooled to about 25°C and crystallized to obtain 22.96 kg of a taurine crude product with a mass content of 94.5%, a water content of 4.5% and an impurity content of 1.0%. A mother solution containing the catalyst was continuously concentrated to 20.52 kg and directly applied in a fresh 35% sodium hydroxyethyl sulfonate aqueous solution, and then corresponding ammonia water and liquid ammonia were supplemented to conduct an ammonolysis reaction directly.

### Example 4

150 kg of 25% ammonia water was pumped into a dissolution kettle, and 0.60 kg of a cobalt acetate catalyst was added and stirred for dissolution. 40 kg of liquid ammonia and 140 kg of a 45% sodium hydroxyethyl sulfonate aqueous solution were added and uniformly stirred to obtain a mixture. After being pressurized to 9.5 MPa by a high pressure pump, the mixture was heated to 195°C by a heat conduction oil preheater first. Then the mixture entered an ammonolysis reactor for a reaction. The ammonolysis reaction was conducted at a temperature of 200°C and a pressure of 9.5 MPa for 40 min to obtain a sodium taurine solution. Then, deamination and dewatering by concentration were conducted to obtain 299.5 kg of a sodium taurine aqueous solution. After sampling for detection, the content of sodium taurine was 19.5%, and the yield of the sodium taurine was calculated as 93.3%.

299.5 kg of the sodium taurine aqueous solution was allowed to flow forward through a 182 L weakly acidic 110 resin at a flow rate of 0.99 BV/h. When the pH of a material liquid at an outlet was close to 8.0, feeding was stopped. Then, the material liquid was washed with deionized water at a forward flow rate of 1.92 BV/h for recycling. The total mass of a collected material was about 653.6 kg, the content of taurine was detected as 7.6%, and the pH of the material liquid was about 7.2. 7.6% of the material was concentrated, cooled to about 25°C and crystallized to obtain 42.05 kg of a taurine crude product with a mass content of 94.0%, a water content of 4.2% and an impurity content of 1.8%. A mother solution containing the catalyst was continuously concentrated to 40.54 kg and directly applied in a fresh 45% sodium hydroxyethyl sulfonate aqueous solution, and then corresponding ammonia water and liquid ammonia were supplemented to conduct an ammonolysis reaction directly.

### Example 5

90 kg of 25% ammonia water was pumped into a dissolution kettle, and 1.02 kg of an iron oxide catalyst was added and stirred for dissolution. 90 kg of liquid ammonia and 210 kg of a 42% sodium hydroxyethyl sulfonate aqueous solution were added and uniformly stirred to obtain a mixture. After being pressurized to 11 MPa by a high pressure pump, the mixture was heated to 195°C by a heat conduction oil preheater first. Then the mixture entered an ammonolysis reactor for a reaction. The ammonolysis reaction was conducted at a temperature of 210°C and a pressure of 11 MPa for 35 min to obtain a sodium taurine solution. Then, deamination and dewatering by concentration were conducted to obtain 337.8 kg of a sodium taurine aqueous solution. After sampling for detection, the content of sodium taurine was 24%, and the yield of the sodium taurine was calculated as 92.5%.

337.8 kg of the sodium taurine aqueous solution was allowed to flow forward through a 309 L weakly acidic 110 resin at a flow rate of 0.93 BV/h. When the pH of a material liquid at an outlet was close to 8.0, feeding was stopped. Then, the material liquid was washed with deionized water at a forward flow rate of 1.7 BV/h for recycling. The total mass of a collected material was about 919.29 kg, the content of taurine was detected as 7.5%, and the pH of the material liquid was about 7.0. 7.5% of the material was concentrated, cooled to about 25°C and crystallized to obtain 59.62 kg of a taurine crude product with a mass content of 94.1%, a water content of 4.3% and an impurity content of 1.6%. A mother solution containing the catalyst was continuously concentrated to 54.51 kg and directly applied in a fresh 42% sodium hydroxyethyl sulfonate aqueous solution, and then corresponding ammonia water and liquid ammonia were supplemented to conduct an ammonolysis reaction directly.

### Example 6

300 kg of 25% ammonia water was pumped into a dissolution kettle, and 0.78 kg of a silver oxide catalyst was added and stirred for dissolution. 40 kg of liquid ammonia and 175 kg of a 40% sodium hydroxyethyl sulfonate aqueous solution were added and uniformly stirred to obtain a mixture. After being pressurized to 9.0 MPa by a high pressure pump, the mixture was heated to 195°C by a heat conduction oil preheater first. Then the mixture entered an ammonolysis reactor for a reaction. The ammonolysis reaction was conducted at a temperature of 195°C and a pressure of 9.0 MPa for 45 min to obtain a sodium taurine solution. Then, deamination and dewatering by concentration were conducted to obtain 318.2 kg of a sodium taurine aqueous solution. After sampling for detection, the content of sodium taurine was 20.5%, and the yield of the sodium taurine was calculated as 93.8%.

318.2 kg of the sodium taurine aqueous solution was allowed to flow forward through a 92 L weakly acidic 196 resin at a flow rate of 0.95 BV/h. When the pH of a material liquid at an outlet was close to 8.0, feeding was stopped. Then, the material liquid was washed with deionized water at a forward flow rate of 1.88 BV/h for recycling. The total mass of a collected material was about 739.85 kg, the content of taurine was detected as 7.5%, and the pH of the material liquid was about 7.1. 7.5% of the material was concentrated, cooled to about 25°C and crystallized to obtain 46.14 kg of a taurine crude product with a mass content of 94.4%, a water content of 4.4% and an impurity content of 1.2%. A mother solution containing the catalyst was continuously concentrated to 46.14 kg and directly applied in a fresh 40% sodium hydroxyethyl sulfonate aqueous solution, and then corresponding ammonia water and liquid ammonia were supplemented to conduct an ammonolysis reaction directly.

### Example 7

200 kg of 25% ammonia water, 0.68 kg of a zinc particle catalyst, 40 kg of liquid ammonia and 170 kg of a 40% sodium hydroxyethyl sulfonate aqueous solution were added and uniformly stirred to obtain a mixture. After being pressurized to 8 MPa, the mixture was heated to 200°C by a heat conduction oil preheater, and then entered an ammonolysis reactor for a reaction for 30 min to obtain a sodium taurine solution. Then, deamination and dewatering by concentration were conducted to obtain 337.80 kg of a sodium taurine aqueous solution. After sampling for detection, the content of sodium taurine was 18.0%, and the yield of the sodium taurine was calculated as 90.0%.

337.80 kg of the sodium taurine aqueous solution was allowed to flow forward through a 159 L weakly acidic 110 resin at a flow rate of 1.2 BV/h. When the pH of a material liquid at an outlet was close to 8.0, feeding was stopped. Then, the material liquid was washed with deionized water at a forward flow rate of 2 BV/h for recycling. The total mass of a collected material was about 795.7 kg, the content of taurine was detected as 6.50%, and the pH of the material liquid was about 7.0. 6.50% of the material was concentrated, cooled to about 25°C and crystallized to obtain 43.77 kg of a taurine crude product with a mass content of 93.1%, a water content of 4.4% and an impurity content of 2.5%.

This example shows that when a catalyst precursor is not subjected to complexation with ammonia water in advance and is directly mixed for a reaction, the ammonolysis reaction may also occur, but the impurity content in the finally obtained product is increased.

Only several embodiments of the present disclosure are expressed in the above examples in more specific and detailed descriptions, but are not construed as limitations of the scope of the invention patent. Therefore, the protection scope of the invention patent shall be defined by the attached claims.

## Claims

1. A method for preparing taurine, **characterized in that** the method comprises the following steps:
(1) subjecting a sodium hydroxyethyl sulfonate aqueous solution to an ammonolysis reaction with ammonia under the action of an ammonia water complex ion catalyst to obtain a reaction solution containing sodium taurine; and
(2) subjecting the reaction solution containing sodium taurine to deamination, dewatering by concentration, acidification and crystallization to obtain the taurine and a mother solution;
and the ammonia water complex ion catalyst is obtained by complexation of ammonia and a catalyst precursor, and the catalyst precursor comprises one or more metal elements selected from chromium, zinc, nickel, cobalt, copper and silver,
wherein in step (1), a method for preparing the ammonia water complex ion catalyst comprises: adding the catalyst precursor to ammonia water, and conducting stirring for dissolution to obtain the ammonia water complex ion catalyst;
and after the ammonia water complex ion catalyst is obtained, the sodium hydroxyethyl sulfonate aqueous solution and liquid ammonia are added for the ammonolysis reaction.

2. The method for preparing taurine according to claim 1, **characterized in that** in step (1), the catalyst precursor is a metal elemental, a metal oxide or an organometallic salt.

3. The method for preparing taurine according to claim 1, **characterized in that** in step (1), the catalyst precursor comprises one or more of chromium trioxide, a zinc powder, nickel acetate, cobalt acetate, copper oxide and silver oxide.

4. The method for preparing taurine according to claim 1, **characterized in that** in step (1), the use amount of the ammonia water complex ion catalyst is 0.5-8 wt% of the sodium hydroxyethyl sulfonate;
the concentration of the sodium hydroxyethyl sulfonate is 10-45 wt%;
and the concentration of the ammonia in the reaction system is 10-30 wt%.

5. The method for preparing taurine according to claim 1, **characterized in that** in step (1), the ammonolysis reaction is conducted at a temperature of 40-210°C;
and the ammonolysis reaction is conducted at a pressure of 0.1-15 MPa.

6. The method for preparing taurine according to claim 1, **characterized in that** in step (2), a flash evaporation method is used as a deamination method;
and the concentration is conducted to increase the concentration of the sodium taurine to 18-25 wt%.

7. The method for preparing taurine according to claim 1, **characterized in that** in step (2), an acidifying agent for acidification is a weakly acidic resin, and the use amount of the weakly acidic resin is 0.5-1 time of the mass of the sodium taurine solution.

8. The method for preparing taurine according to claim 1, **characterized in that** in step (2), the crystallization is cooling crystallization, and the crystallization is conducted at a temperature of 20-40°C for 2-8 h.

9. The method for preparing taurine according to claim 1, **characterized in that** in step (2), the mother solution is concentrated and then applied;
and a specific application method comprises:
mixing the concentrated mother solution with ammonia water, liquid ammonia and a fresh sodium hydroxyethyl sulfonate aqueous solution for a next batch of ammonolysis reaction.

## Patentansprüche

1. Verfahren zur Herstellung von Taurin, **dadurch gekennzeichnet, dass** das Verfahren die folgenden Schritte umfasst:
(1) Unterziehen einer wässrigen Natriumhydroxyethylsulfonat-Lösung einer Ammonolyse-Reaktion mit Ammoniak unter der Einwirkung eines Ammoniakwasser-Komplexionenkatalysators, um eine Natriumtaurin enthaltende Reaktionslösung zu erhalten; und
(2) Unterziehen der Natriumtaurin enthaltenden Reaktionslösung einer Desaminierung, Entwässerung durch Konzentration, Ansäuerung und Kristallisation, um das Taurin und eine Mutterlösung zu erhalten;
und der Ammoniakwasser-Komplexionenkatalysator wird durch Komplexierung von Ammoniak und einem Katalysatorvorläufer erhalten, und der Katalysatorvorläufer umfasst ein oder mehrere Metallelemente, ausgewählt aus Chrom, Zink, Nickel, Kobalt, Kupfer und Silber,
wobei in Schritt (1) ein Verfahren zur Herstellung des Ammoniakwasser-Komplexionenkatalysators umfasst: Zugeben des Katalysatorvorläufers zu Ammoniakwasser und Durchführen von Rühren zur Auflösung, um den Ammoniakwasser-Komplexionenkatalysator zu erhalten;
und nachdem der Ammoniakwasser-Komplexionenkatalysator erhalten wurde, werden die wässrige Natriumhydroxyethylsulfonat-Lösung und flüssiges Ammoniak für die Ammonolyse-Reaktion zugegeben.

2. Verfahren zur Herstellung von Taurin nach Anspruch 1, **dadurch gekennzeichnet, dass** in Schritt (1) der Katalysatorvorläufer ein elementares Metall, ein Metalloxid oder ein metallorganisches Salz ist.

3. Verfahren zur Herstellung von Taurin nach Anspruch 1, **dadurch gekennzeichnet, dass** in Schritt (1) der Katalysatorvorläufer eines oder mehrere von Chromtrioxid, ein Zinkpulver, Nickelazetat, Kobaltazetat, Kupferoxid und Silberoxid umfasst.

4. Verfahren zur Herstellung von Taurin nach Anspruch 1, **dadurch gekennzeichnet, dass** in Schritt (1) die Einsatzmenge des Ammoniakwasser-Komplexionenkatalysators 0,5-8 Gew.-% des Natriumhydroxyethylsulfonats beträgt;
die Konzentration des Natriumhydroxyethylsulfonats 10-45 Gew.-% beträgt;
und die Konzentration des Ammoniaks in dem Reaktionssystem 10-30 Gew.-% beträgt.

5. Verfahren zur Herstellung von Taurin nach Anspruch 1, **dadurch gekennzeichnet, dass** in Schritt (1) die Ammonolyse-Reaktion bei einer Temperatur von 40-210 °C durchgeführt wird;
und die Ammonolyse-Reaktion bei einem Druck von 0,1-15 MPa durchgeführt wird.

6. Verfahren zur Herstellung von Taurin nach Anspruch 1, **dadurch gekennzeichnet, dass** in Schritt (2) ein Flash-Verdampfungsverfahren als Desaminierungsverfahren verwendet wird;
und die Konzentrierung durchgeführt wird, um die Konzentration des Natriumtaurins auf 18-25 Gew.-% zu erhöhen.

7. Verfahren zur Herstellung von Taurin nach Anspruch 1, **dadurch gekennzeichnet, dass** in Schritt (2) ein Ansäuerungsmittel zur Ansäuerung ein schwach saures Harz ist und die verwendete Menge des schwach sauren Harzes 0,5-1 mal die Masse der Natriumtaurinlösung ist.

8. Verfahren zur Herstellung von Taurin nach Anspruch 1, **dadurch gekennzeichnet, dass** in Schritt (2) die Kristallisation eine Kühlkristallisation ist und die Kristallisation bei einer Temperatur von 20-40 °C für 2-8 h durchgeführt wird.

9. Verfahren zur Herstellung von Taurin nach Anspruch 1, **dadurch gekennzeichnet, dass** in Schritt (2) die Mutterlösung konzentriert und dann appliziert wird;
und ein spezifisches Applikationsverfahren umfasst:
Mischen der konzentrierten Mutterlösung mit Ammoniakwasser, flüssigem Ammoniak und einer frischen wässrigen Natriumhydroxyethylsulfonat-Lösung für eine nächste Charge der Ammonolyse-Reaktion.

## Revendications

1. Procédé de préparation de taurine, **caractérisé en ce que** le procédé comprend les étapes suivantes :
(1) le fait de soumettre une solution aqueuse d'hydroxyéthylsulfonate de sodium à une réaction d'ammonolyse avec de l'ammoniac sous l'action d'un catalyseur ionique complexe d'eau ammoniacale pour obtenir une solution de réaction contenant de la taurine de sodium ; et (2) le fait de soumettre la solution de réaction contenant de la taurine de sodium à une désamination, à une déshydratation par concentration, à une acidification et à une cristallisation pour obtenir la taurine et une solution mère ; et le catalyseur ionique complexe d'eau ammoniacale est obtenu par complexation d'ammoniac et d'un précurseur de catalyseur, et le précurseur de catalyseur comprend un ou plusieurs éléments métalliques choisis parmi le chrome, le zinc, le nickel, le cobalt, le cuivre et l'argent, dans lequel à l'étape (1), un procédé de préparation du catalyseur ionique complexe d'eau ammoniacale comprend : l'ajout du précurseur de catalyseur à l'eau ammoniacale, et la réalisation d'une agitation pour dissolution afin d'obtenir le catalyseur ionique complexe d'eau ammoniacale ; et après l'obtention du catalyseur ionique complexe d'eau ammoniacale, la solution aqueuse d'hydroxyéthylsulfonate de sodium et de l'ammoniac liquide sont ajoutés pour la réaction d'ammonolyse.

2. Procédé de préparation de taurine selon la revendication 1, **caractérisé en ce que**, à l'étape (1), le précurseur de catalyseur est un élément métallique, un oxyde métallique ou un sel organométallique.

3. Procédé de préparation de taurine selon la revendication 1, **caractérisé en ce que**, à l'étape (1), le précurseur de catalyseur comprend un ou plusieurs éléments parmi le trioxyde de chrome, une poudre de zinc, l'acétate de nickel, l'acétate de cobalt, l'oxyde de cuivre et l'oxyde d'argent.

4. Procédé de préparation de taurine selon la revendication 1, **caractérisé en ce que**, à l'étape (1), la quantité d'utilisation du catalyseur ionique complexe d'eau ammoniacale est de 0,5 à 8 % en poids de l'hydroxyéthylsulfonate de sodium ; la concentration de l'hydroxyéthylsulfonate de sodium est de 10 à 45 % en poids ;
et la concentration de l'ammoniac dans le système de réaction est de 10 à 30 % en poids.

5. Procédé de préparation de taurine selon la revendication 1, **caractérisé en ce que**, à l'étape (1), la réaction d'ammonolyse est réalisée à une température de 40 à 210 °C ; et la réaction d'ammonolyse est réalisée à une pression de 0,1 à 15 MPa.

6. Procédé de préparation de taurine selon la revendication 1, **caractérisé en ce que**, à l'étape (2), un procédé d'évaporation éclair est utilisé en tant que procédé de désamination ; et la concentration est réalisée pour augmenter la concentration de la taurine de sodium à 18 à 25 % en poids.

7. Procédé de préparation de taurine selon la revendication 1, **caractérisé en ce que**, à l'étape (2), un agent acidifiant pour l'acidification est une résine faiblement acide, et la quantité d'utilisation de la résine faiblement acide est de 0,5 à 1 fois la masse de la solution de taurine de sodium.

8. Procédé de préparation de taurine selon la revendication 1, **caractérisé en ce que**, à l'étape (2), la cristallisation est une cristallisation par refroidissement, et la cristallisation est réalisée à une température de 20 à 40 °C pendant 2 à 8 heures.

9. Procédé de préparation de taurine selon la revendication 1, **caractérisé en ce que**, à l'étape (2), la solution mère est concentrée puis appliquée ; et un procédé d'application spécifique comprend : le mélange de la solution mère concentrée avec de l'eau ammoniacale, de l'ammoniac liquide et une solution aqueuse fraîche d'hydroxyéthylsulfonate de sodium pour un lot suivant de réaction d'ammonolyse.
